(19) Europäisches Patentamt
European Patent Office
Office européen des brevets

(11) Publication number: 0 319 061 A1

(12) **EUROPEAN PATENT APPLICATION**

(21) Application number: 88202498.7

(22) Date of filing: 09.11.88

(51) Int. Cl.4: A61K 31/55 , A61K 31/445

(30) Priority: 28.11.87 NL 8702859

(43) Date of publication of application: 07.06.89 Bulletin 89/23

(84) Designated Contracting States: AT BE CH DE FR GB IT LI NL SE

(71) Applicant: AKZO N.V.
Velperweg 76
NL-6824 BM Arnhem(NL)

(72) Inventor: de Boer, Thijs
Kouterstraat 9
NL-5345 AR Oss(NL)
Inventor: Ruigt, Gerardus Stephanus Franciscus
Monsterstraat 6
NL-5314 EB Oss(NL)

(74) Representative: Hermans, Franciscus G.M. et al
Patent Department AKZO N.V. Pharma Division P.O. Box 20
NL-5340 BH Oss(NL)

(54) **Medicament with antiemetic action.**

(57) The present invention relates to the use of a group of tetracyclic compounds of the general formula I

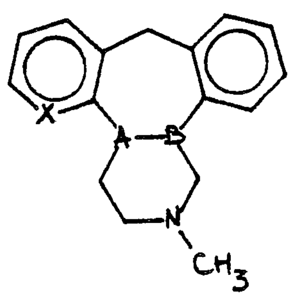

I

or a pharmaceutically acceptable salt thereof, in which X represents carbon (C) or nitrogen (N) and A-B represents an N-CH or a C=C bond, for the preparation of a medicament with antiemetic action.

EP 0 319 061 A1

## Medicament with antiemetic action

The present invention relates to a medicament with antiemetic action. More particularly, the invention relates to the use of a group of tetracyclic compounds for the preparation of a medicament to treat stomach discharge disorders, not being of psycho-somatic nature.

The relevant group of antiemetically active compounds has the general formula:

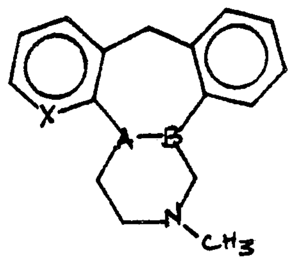

I

or a pharmaceuticaly acceptable salt thereof, in which X represents carbon (C) or nitrogen (N) and A-B represents an N-CH or a C=C bond.

The above tetracyclic compounds having the formula I are known. They are specifically described in the US Patent Specifications USP 3,534,041, USP 4,002,632 and USP 4,062,848, for their antihistamine, antiserotonin and antidepressant properties.

Moreover, it appears form the European Patent application 126,343 that some compounds having formula I (e.g. mianserin, X = C; A-B = N-CH) have the property of stopping or preventing diarrhoea.

Due to their antidepressant activity compounds of formula I may secondary reduce psycho-somatic complaints, like nausea and vomiting. For instance, A. Bjertnaes et al. (Acta psychiat. Scand. (1982), 66, 199) report that mianserin causes less nausea and vomiting than placebo treated patients suffering from primary "free-floating" anxiety, whereas D. Costa et al. (ibid. (1985), 72. Suppl. 320, 85) remark that mianserin does not induce nausea/vomiting in depressed patients. These effects (or lack of effects) on psycho-somatic nausea/vomiting are completely in line with the expectation of efficacy of anti-depressant drugs, and have no relation with antiemetic activity.

Surprisingly, it has now, however, been found that the compounds I exhibit a powerful antiemetic action, specifically in the case of cancer patients to whom high dosages of cytostatics (e.g. cis-platinum) or radiotherapy have been administered. These patients, nearly without exception, suffer from violent nausea/vomiting as side-effect of the cytostatic treatment. The compounds I are therefore extremely suitable for the treatment or prevention of stomach discharge disorders, not being of psycho-somatic nature.

Said antiemetic action occurs immediately after administration and consequently differs essentially from the antidepressive action already mentioned previously which only occurs after one or two weeks of chronic administration and sometimes even after a still longer time.

Compounds according to formula I which are to be preferred for use as an antiemetic are (in order or preference):

(a) 1,2,3,4,10,14b-hexahydro-2-methyldibenzo[c,f]-pyrazino[1,2-a]azepine and salts thereof (X = C; A-B = N-CH);

(b) (+)-1,2,3,4,10,14b-hexahydro-2-methylpyrazino[2,1-a]pyrido[2,3-c][2]benzazepine and salts thereof (X = N; A-B = N-CH);

(c) rac-1,2,3,4,10,14b-hexahydro-2-methylpyrazino[2,1-a]pyrido[2,3-c] [2]benzazepine and salts thereof (X = N; A-B = N-CH);

(d) N-methyl-1,2,3,4-tetrahydro-9H-dibenzo[a,c]pyridino[3,4-c]cycloheptatriene and salts thereof (X = C; A-B = C = C).

Here salts are understood to mean the pharmaceutically acceptable salts which are obtained by reaction of the free base according to formula I with a suitable organic or inorganic acid. Suitable salts are, for example, the hydrochloride, fumarate, succinate and citrate.

The compounds according to the invention may be administered both locally, enterally and parenterally. As suitable enteral dosage forms, the oral, but above all the anal and vaginal dosage forms and the dosage

form via ear, nose or eye, are eligible. For this purpose, the antiemetically active compound according to formula I is mixed with suitable auxiliary and/or carrier substances and then processed into a form which is suitable for oral, ophthalmic, otic, nasal, anal or vaginal administration, such as, for example, in the form of a spray, eye or ear drop, pill, tablet, capsule, rectal suppository or vaginal suppository. For parenteral administration, the antiemetically active compound is dissolved, suspended or emulsified in a suitable liquid and then filled into a dosage form suitable for injection.

For local administration, a transdermal plaster is particularly suitable.

The compounds according to formula I are administered in a daily dosage of 0.05 mg - 5 mg/kg of body weight and, more particularly, in a daily dosage of 0.5 mg - 2 mg/kg of body weight.

For human administration, preference is given, in particular, to a daily dosage of 30 mg to 150 mg for oral and local administration, and from 10 to 50 mg for use in injection preparations or in rectal suppositories, vaginal suppositories, eye, nose and ear drops.

Some compounds according to formula I have a chiral carbon atom so that these compounds may occur as a racemate, but also as the separate (+) or (-) enantiomers. Both enantiomers and the racemate belong to the invention.

## Examples

The following active substances have been used in the present formulations:

A: 1,2,3,4,10,14b-hexahydro-2-methyldibenzo[c,f]-pyrazino [1,2-a]azepine hydrochloride, and

B: 1,2,3,4,10,14b-hexahydro-2-methylpyrazino[2,1-a]pyrido[2,3-c][2]benzazepine.

Tablet with fraction line

| | |
|---|---|
| Active substance B | 30 mg |
| Starch | 30 mg |
| Hydroxypropylcellulose | 6 mg |
| Magnesium stearate | 1.5 mg |
| Lactose | to make 300 mg |

Injection preparation

| | |
|---|---|
| Ative substance B | 9 mg |
| NaOH/HCl | to pH 5 |
| Mannitol | 25 mg |
| Water | to make 1.0 ml |

Vaginal suppository and rectal suppository

| | |
|---|---|
| Active substance A | 14 mg |
| Whitepsol 558 | 1500 mg |

The wax is melted, the active substance is dissolved therein, after which the mixture is cooled in the appropriate mould.

Ophthalmic solution

| | |
|---|---|
| Active substance B | 4.5 g |
| Polyvinyl alcohol | 7.0 g |
| $Na_2HPO_4$ | 3.8 g |
| $NaH_2PO_4$ | 0.8 g |
| Chlorobutanol | 2.5 g |
| NaCl | Until isotonic |
| Water | To make 500 ml |

Otic preparation

| | |
|---|---|
| Active subtance B | 2.25 g |
| Benzyl alcohol | 10.0 g |
| NaCMC | 0.025 g |
| Propylene glycol | 25.0 g |
| NaCl | Until isotonic |
| NaOH/HCl | To pH 5 |
| Water | To make 250 ml |

Nasal preparation

| | |
|---|---|
| Active substance B | 90 mg |
| Benzalkonium chloride | 0.001 mg |
| EDTA | 0.01 mg |
| NaOH/HCl | To pH 5 |
| Mannitol | Until isotonic |
| Water | To make 10 ml |

## Claims

1. Pharmaceutical composition for the treatment of stomach discharge disorders not being of psychosomatic nature which comprises as active constituent a substance with the general formula I

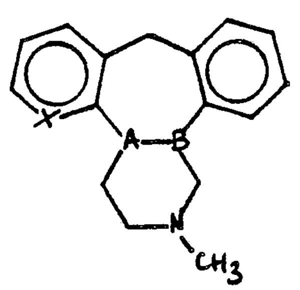

I

or a pharmaceutically acceptable salt thereof, in which X represents carbon (C) or nitrogen (N) and A-B represents either an N-CH bond or a C=C bond.

2. Use of a substance with the general formula I

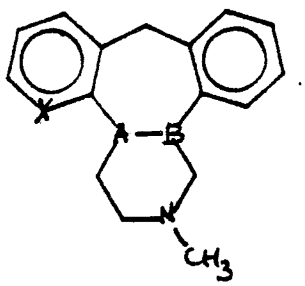

I

or a pharmaceutically acceptable salt thereof, in which X represents carbon (C) or nitrogen (N) and A-B represents either an N-CH bond or a C=C bond, for the preparation of a medicament with antiemetic action, to treat stomach discharge disorders which are not of psycho-somatic nature.

3. Method for the treatment of patients with stomach discharge disorders not being of psycho-somatic nature by administering to said patients an antiemetically effective dose of a compound having formula I or a salt thereof as defined in claim 1.

European Patent Office

# PARTIAL EUROPEAN SEARCH REPORT

which under Rule 45 of the European Patent Convention shall be considered, for the purposes of subsequent proceedings, as the European search report

Application number
EP 88 20 2498

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | ACTA PSYCHIATR. SCAND. , vol. 72, suppl. 320, 1985, pages 85-92<br>D. COSTA et al.: "Efficacy and safety of mianserin in the treatment of depression of women with cancer"<br>* Abstract, last sentence; page 86, left-hand column, paragraph 4; page 89, right-hand column, lines 11-13; page 90, table 7; page 91, left-hand column, paragraphs 2,3 *<br>-- | 1,2 | A 61 K 31/55<br>A 61 K 31/445 |
| X,D | ACTA PSYCHIATR. SCAND., vol. 66, no. 3, 1982, pages 199-207<br>A. BJERTNAES et al. "A multicentre placebo-controlled trial comparing the efficacy of mianserin and chlordiazepoxide in general practice patients with primary anxiety"<br>* Summary, last paragraph; page 204, table 5 and last paragraph, page 206, lines 14,15 *<br>-- ./. | 1,2 | |

TECHNICAL FIELDS SEARCHED (Int. Cl.4)

A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2
Claims searched incompletely:
Claims not searched: 3
Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 01-12-1988 | THEUNS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82

European Patent Office

# PARTIAL EUROPEAN SEARCH REPORT

Application number
EP 88 20 2498
-2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | THE MERCK MANUAL OF DIAGNOSIS AND THERAPY, 14th Ed. 1982, Merck & Co. Inc., Rahway, N.J. US, pages 2341-2344<br>* Page 2342, "Antihistamines" *<br>-------- | 1,2 | |
| | | | TECHNICAL FIELDS SEARCHED (Int. Cl.4) |
| | | | |